(19) Europäisches Patentamt
European Patent Office
Office européen des brevets

(11) Publication number: 0 196 016 B1

# (12) EUROPEAN PATENT SPECIFICATION

(45) Date of publication of patent specification: 22.08.90

(51) Int. Cl.$^5$: C 07 D 213/74, A 61 K 31/44

(21) Application number: 86103791.9

(22) Date of filing: 20.03.86

## (54) 3-nitropyridine-derivatives, process for their preparation and pharmaceutical composition containing them.

(30) Priority: 27.03.85 US 716885

(43) Date of publication of application:
01.10.86 Bulletin 86/40

(45) Publication of the grant of the patent:
22.08.90 Bulletin 90/34

(84) Designated Contracting States:
CH DE FR GB IT LI NL

(56) References cited:

JOURNAL OF MEDICINAL CHEMISTRY, vol. 16, no. 5, May 1973, pages 520-524; M. ISRAEL et al.: "Pyrido3,2-grpteridines. 2. Synthesis and growth-inhibitory evaluation of some 10-substituted 3H,10H-2,4-dioxopyrido3,2-grpteridines(9-azaisoalloxazines)"

(73) Proprietor: MERCK & CO. INC.
126, East Lincoln Avenue P.O. Box 2000
Rahway New Jersey 07065-0900 (US)

(72) Inventor: Saari, Walfred S.
1740 Wagon Wheel Lane
Lansdale, PA. 19446 (US)

(74) Representative: Blum, Rudolf Emil Ernst et al
c/o E. Blum & Co Patentanwälte Vorderberg 11
CH-8044 Zürich (CH)

(56) References cited:
JOURNAL OF MEDICINAL CHEMISTRY, vol. 28, no. 4, April 1985, pages 467-471, American Chemical Society; D.M. HOUSTON et al.: "Potential inhibitors of S-adenosylmethionine-dependent methyltransferases. 8. Molecular dissections of carbocyclic 3-deazaadenosine as inhibitors of S-adenosylhomocysteine hydrolase"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 196 016 B1

Courier Press, Leamington Spa, England.

## Description

Background of the Invention

The present invention relates to two new substances which are 3-nitropyridine compounds having a 2,3-dihydroxy-1-propylamino substituent attached at the position 4 or 6 and salts of said compounds.

Said new compounds as well as a related compound which is already described as intermediate product in the prior art have the ability to enhance the therapeutic effect of radiation.

At the present time, certain other unrelated compounds are in experimental clinical use as radiation sensitizers. However, these compounds — for example, metronidazole and misonidazole — suffer from the drawback that they also cause neurotoxicity which limits their usefulness.

Description of the Prior Art

In the Journal of Medicinal Chemistry, vol. 16, no. 5, pages 520—524, 1973, there are described pyrido[3,2-g]pteridines. Said compounds, accordingly, have a ring system of three condensed rings, one of which is a pyridine ring and they are stated to have an antitumor activity. According to the reaction scheme outlined on page 521 of said publication the pyrido[3,2-g]pteridines are prepared by using as starting material the 2-chloro-3-nitropyridine which is reacted with an amine of formula

$$RNH_2,$$

to yield the corresponding 2-amino-3-nitro-pyridine, which is thereafter reduced yielding a corresponding 2,3-diamino-pyridine which is submitted to further reactions in order to produce the desired final products. In table I there is mentioned the corresponding 2-amino-3-nitropyridine intermediate product in which the radical R of the amino group has the structure

$$—CH_2—CH(OH)—CH_2—OH.$$

Said intermediate product, accordingly, corresponds to formula III

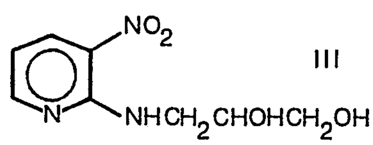

III

It was now unexpectedly found out that said prior art compound of formula III, as well as corresponding new compounds in which the stated amino substituent is not in the position 2, but attached at the position 4 or 6 of the pyridine nucleus have the activity of enhancing the therapeutic effect of radiation.

Description of the Invention

One subject of the present invention are new 3-nitropyridine compounds of formula I

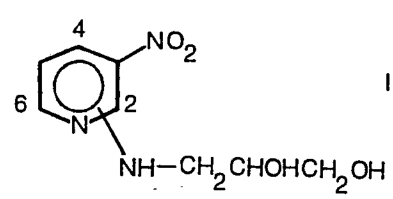

I

in which the $—NHCH_2CHOHCH_2OH$ group is attached at position 4 or 6 of the pyridine ring or salts of said compounds. One of the claimed compounds of formula I is the 4-(2,3-dihydroxy-1-propylamino)-3-nitropyridine which has the following formula Ia

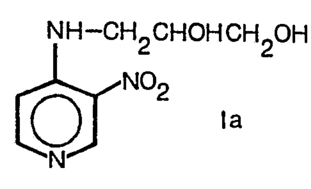

Ia

A further object of the present invention is a process for the preparation of the 3-nitropyridine compounds of formula I

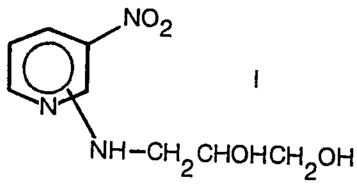

I

and said process is characterized in that a 3-nitropyridine, having in the position 4 or the position 6 a leaving group, is reacted with an amine of formula II

$$H_2NCH_2CHOHCH_2OH \qquad II$$

and the resulting product of formula I is isolated in the free form or as salt thereof.

According to a preferred embodiment of said process in the used starting material the leaving group is a halo atom, preferably a chlorine atom. The starting material, accordingly, is the corresponding chloro-3-nitropyridine of formula

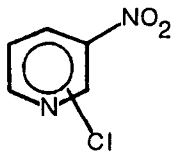

wherein the chloro substituent is in the position 4 or 6. The reaction is preferably performed in a suitable solvent such as a lower aliphatic alcohol, or a polar aprotic solvent such as dimethyl-formamide, dimethylsulfoxide, or others such as tetrahydrofuran, glyme, diglyme, tetramethylurea, chloroform, or methylene chloride.

The treatment of the starting material with the amine of formula II is preferably performed in sufficient base to neutralize the hydrogen chloride formed. The reaction temperature is not critical and may vary from 0—100°C, preferably from about 0—25°C for a period of from 1—24 hours.

Suitable bases employed to neutralize the hydrogen chloride formed are tertiary amines such as triethylamine and pyridine. If desired, the neutralizing base may be supplied by using excess amine reactant. Inorganic bases such as alkali metal bicarbonates, carbonates and hydroxides may also be employed.

The product is recovered in substantially pure form by removal of solvent by evaporation under reduced pressure and the residue containing the product is chromatographed and crystallized from suitable solvents.

A further object of the present invention is a pharmaceutical composition for enhancing the therapeutic effect of radiation and said composition is characterized in that it contains as pharmaceutically effective ingredient an inventive compound of formula I or a pharmaceutically acceptable salt thereof.

It was furthermore found out that the corresponding prior art compound of formula III, in which the amino substituent of the stated formula is in the position 2 and not in the position 4 or 6, has as well the pharmaceutical activity of enhancing the therapeutic effect of radiation.

A further object of the present invention is therefore the use of the 2-(2,3-dihydroxy-1-propylamino)-3-nitropyridine of formula III or of a pharmaceutical salt thereof for the preparation of a pharmaceutical composition for enhancing the therapeutic effect of radiation.

Pharmaceutical compositions which contain as active ingredient the inventive compounds of formula I or the prior art compound of formula III or pharmaceutically acceptable salts of the compounds of formula I or III, can be used to treat human patients or domestic animals which are submitted to a radiation treatment of malignant diseases. Corresponding pharmaceutical compositions can be administered orally or intravenously.

The dosage in which said pharmaceutical compositions are employed depends on the radiation protocol for each individual patient. In protocols where the radiation dose is divided into a large number of fractions, the drug can be administered at intervals in the schedule and not necessarily with each radiation treatment. It should be noted that the compounds of the present invention are not intended for chronic administration. In general, the drug is administered from 10 minutes to 5 hours prior to the radiation treatment in a dosage amount of between 0.25 to about 4.0 grams per square meter of body surface.

The dosage range given is the effective dosage range and the decision as to the exact dosage used must be made by the administering physician based on his judgement of the patient's general physical condition. In determining the dose for the individual patient, the physician may begin with an initial dose of 0.25 g/square meter of body surface to determine how well the drug is tolerated and increase the dosage with each succeeding radiation treatment, observing the patient carefully for any drug side effect. The competition to be administered is an effective amount of the active compound and a pharmaceutical carrier for said active compound.

Example 1

4-(2,3-Dihydroxy-1-propylamino)-3-nitropyridine

A solution of 4-chloro-3-nitropyridine (1.1 g, 6.94 mmol) and 3-amino-1,2-propanediol (1.22 g, 13.4 mmol) in isopropanol (50 ml) was stirred at 20—25°C for 20 hours and then concentrated under reduced pressure. Flash chromatography of the residue over silica gel and elution with 10% MeOH—90% $CHCl_3$ gave pure 4-(2,3-dihydroxy-1-propylamino)-3-nitropyridine (500 mg, 33.8%). An analytically pure sample, m.p. 131—35°C, was obtained upon recrystallization from MeOH-EtOAc-hexane.

Example 2

6-(2,3-Dihydroxy-1-propylamino)-3-nitropyridine

A solution of 6-chloro-3-nitropyridine (3.22 g, 20.3 mmol), 3-amino-1,2-propanediol (1.85 g, 20.3 mmol) and triethylamine (2.05 g, 20.3 mmol) in isopropanol (80 ml) was stirred at 20—25°C for 18 hours and then at reflux for 6 hours. After concentrating under reduced pressure, the residue was flash chromatographed over silica gel. Elution with 5% MeOH—95% $CHCl_3$ and recrystallization from EtOAc-hexane afforded the analytically pure product named in the title.

Example 3

The prior art compound of formula III, i.e. the 2-(2,3-dihydroxy-1-propylamino)-3-nitropyridine was prepared according to the process described in example 2. As starting material however 2-chloro-3-nitropyridine was used instead of the 6-chloro-3-nitropyridine.

According to said process 2,64 g (61%) of the 2-(2,3-dihydroxy-1-propylamino)-3-nitropyridine were recovered and said product had a melting point of 95.5—98.0°.

**Claims**

1. 3-nitropyridine compounds of the formula I

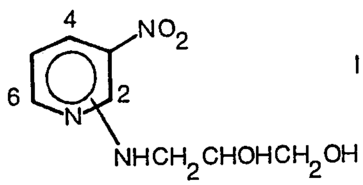

I

wherein the $-NHCH_2CHOHCH_2OH$ substituent is attached at position 4 or 6, or salts of the compounds of formula I.

2. A compound according to claim 1 which is 4-(2,3-dihydroxy-1-propylamino)-3-nitropyridine having the following formula Ia

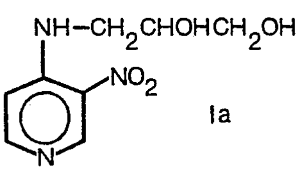

Ia

3. Process for the preparation of the 3-nitropyridine compounds of formula I

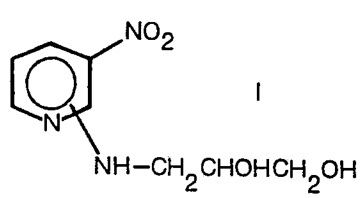

I

according to claim 1, characterized in that a 3-nitro-pyridine, having in the position 4 or the position 6 a leaving group is reacted with an amine of formula II

$$H_2NCH_2CHOHCH_2OH \qquad II$$

and the resulting product of formula I is isolated in the free form or as salt thereof.

4. Process according to claim 3, characterized in that in the starting material the leaving group is a haloatom, preferably chloride, and that the reaction is performed in the presence of a base.

5. Pharmaceutical composition for enhancing the therapeutic effect of radiation, characterized in that it contains as pharmaceutically effective ingredient a compound of formula I according to claim 1 or a pharmaceutically acceptable salt thereof.

6. Pharmaceutical composition according to claim 5, characterized in that it furthermore contains a pharmaceutically acceptable carrier.

7. Pharmaceutical composition according to claim 5 or 6, characterized in that it contains as pharmaceutically effective ingredient a compound of formula Ia according to claim 2.

8. Use of the 2-(2,3-dihydroxy-1-propylamino)-3-nitropyridine of formula III

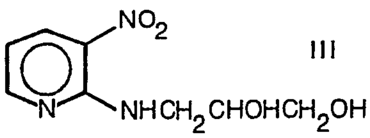

III

or of a pharmaceutically acceptable salt of said compound of formula III for the preparation of a pharmaceutical composition for enhancing the therapeutic effect of radiation.

## Patentansprüche

1. 3-Nitropyridinverbindungen der Formel I

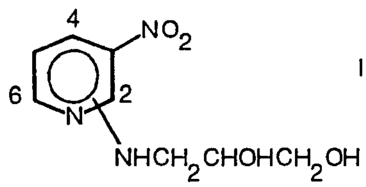

I

worin der —$NHCH_2CHOHCH_2OH$-Substituent in der 4- oder 6-Stellung gebunden ist, oder Salze der Verbindungen der Verbindungen der Formel I.

2. Verbindung nach Anspruch 1, welche 4-(2,3-Dihydroxy-1-propylamino)-3-nitropyridin der folgenden Formel Ia ist

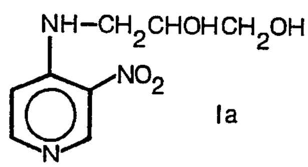

Ia

3. Verfahren zur Herstellung der 3-Nitropyridinverbindungen der Formel I

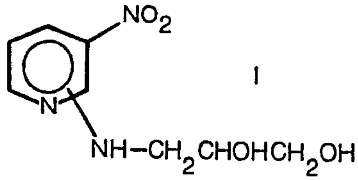

I

nach Anspruch 1, dadurch gekennzeichnet, dass ein 3-Nitropyridin mit einer austretenden Gruppe in der 4-Stellung oder der 6-Stellung mit einem Amin der Formel II umgesetzt wird

$$H_2NCH_2CHOHCH_2OH \quad II$$

und das resultierende Produkt der Formel I in freier Form oder als ihr Salz isoliert wird.

4. Verfahren nach Anspruch 3, dadurch gekennzeichnet, dass im Austauschmaterial die austretende Gruppe ein Halogenatom, vorzugsweise Chlorid, ist und dass die Reaktion in Gegenwart einer Base durchgeführt wird.

5. Pharmazeutische Zusammensetzung zur Stärkung des therapeutischen Effekts von Strahlung, dadurch gekennzeichnet, dass sie als pharmazeutisch wirksamen Bestandteil eine Verbindung der Formel I nach Anspruch 1 oder ein pharmazeutisch annehmbares Salz davon enthält.

6. Pharmazeutische Zusammensetzung nach Anspruch 5, dadurch gekennzeichnet, dass sie weiterhin einen pharmazeutisch annehmbaren Träger enthält.

7. Pharmazeutische Zusammensetzung nach Anspruch 5 oder 6, dadurch gekennzeichnet, dass sie als pharmazeutisch wirksamen Bestandteil eine Verbindung der Formel Ia nach Anspruch 2 enthält.

8. Verwendung von 2-(2,3-Dihydroxy-1-propylamino)-3-nitropyridin der Formel III

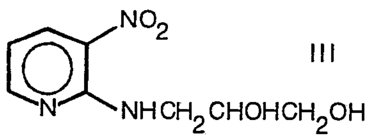

III

oder eines pharmazeutisch annehmbaren Salzes der Verbindung der Formel III zur Herstellung einer pharmazeutischen Zusammensetzung zur Verstärkung des therapeutischen Effekts von Strahlung.

**Revendications**

1. Dérivés de 3-nitropyridine de formule I

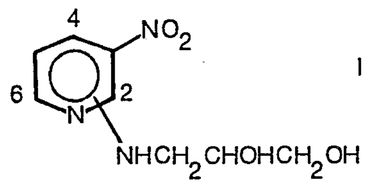

I

dans laquelle le substituant $—NHCH_2CHOHCH_2OH$ est fixé sur la position 4 ou 6 ou les sels des composés de formule I.

2. Composé selon la revendication 1 qui est la 4-(2,3-dihydroxy-1-propylamino)-3-nitropyridine répondant à la formule Ia suivante

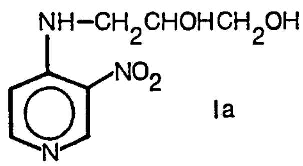

Ia

3. Procédé pour la préparation des dérivés de 3-nitropyridine de formule I

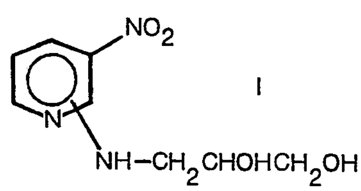

I

selon la revendication 1, caractérisé en ce que l'on fait réagir une 3-nitropyridine ayant un groupe labile dans la position 4 ou la position 6 avec une amine de formule II

$$H_2NCH_2CHOHCH_2OH \qquad II$$

et on isole le produit obtenu de formule I sous la forme libre ou sous forme d'un sel.

4. Procédé selon la revendication 3, caractérisé en ce que, dans la matière de départ, le groupe labile est un atome d'halogène, de préférence de chlore, et la réaction est effectuée en présence d'une base.

5. Composition pharmaceutique pour accroître l'effet thérapeutique des rayonnements, caractérisée en ce qu'elle contient comme ingrédient pharmaceutiquement efficace un composé de formule I selon la revendication 1 ou un sel pharmaceutiquement acceptable de celui-ci.

6. Composition pharmaceutique selon la revendication 5, caractérisé en ce qu'elle contient de plus un véhicule pharmaceutiquement acceptable.

7. Composition pharmaceutiquement selon la revendication 5 ou 6, caractérisée en ce qu'elle contient comme ingrédient pharmaceutiquement efficace un composé de formule Ia selon la revendication 2.

8. Utilisation de la 2-(2,3-dihydroxy-1-propylamino)-3-nitropyridine de formule III

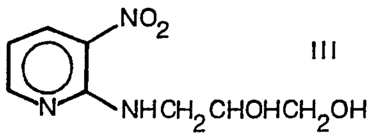

III

ou d'un sel pharmaceutiquement acceptable dudit composé de formule III pour la préparation d'une composition pharmaceutique pour accroître l'effet thérapeutique des rayonnements.